# EUROPEAN PATENT SPECIFICATION

(11) **EP 1 524 966 B1**
(45) Date of publication and mention of the grant of the patent: **25.11.2009**
(21) Application number: 02754972.4
(22) Date of filing: 19.07.2002
(51) Int. Cl.: A61K 9/50, A61K 31/70

(54) **TASTE MASKED ORAL COMPOSITION OF TELITHROMYCIN**
GESCHMACKSMASKIERTE ORALE ZUBEREITUNG VON TELITHROMYCIN
COMPOSITION ORALE DE TELITHROMYCINE POSSEDANT UN GOUT AGREABLE

(43) Date of publication of application: 27.04.2005
(73) Proprietor: Aventis Pharma S.A., 92165 Antony Cédex (FR)
(72) Inventor: BECOURT, Philippe, F-91300 MASSY (FR); BOLTRI, Luigi, I-20041 Milan (IT); CIOLOCA, Nicoletta, F-93270 Sevran (FR); DE LUIGI BRUSCHI, Stefano, I-20146 Milan (IT); MAPELLI, Luigi, Giovanni, I-20100 Milan (IT); RABAGLIA, Leonardo, I-43100 Parme (IT); SCHWABE, Detlev, F-92380 Garches (FR)
(86) International application number: PCT/EP2002/008670
(87) International publication number: WO 2004/009059

(56) References cited:
- EP-A- 0 293 885
- WO-A-01/14393
- US-A- 5 635 485
- US-A- 5 945 124

## Description

The invention relates to an oral composition comprising telithromycin that have taste masking properties, and the process for the preparation thereof.

Telithromycin is an already known antibacterial active described in EP 680,967. Unfortunately it has an unpleasant taste and therefore it cannot be formulated in a simple oral composition, mainly for paediatric use, and so far it has not be found how to overcome the problem of preparing a composition that could be acceptable.

The international application WO 01/14393 describes spherical agglomerates of telithromycin which are obtained by direct transformation of cristals into spherical forms. These agglomerates have a size of between 30 and 400µm and preferentially 80 to 150µm. The described agglomerates are used to prepare micro-capsules for the preparation of oral suspensions, with using a coacervation process. Unfortunately when using the general methods of micro-encapsulation and coacervation it is impossible to obtain a composition with sufficient properties of taste masking.

It has now be found that using specific components it is possible to prepare a composition of telithromycin which taste is enough masked to be acceptable on an organoleptic point of view and where the suspension after reconstitution has good stability properties and acceptable bioavailability.

According to the invention the composition comprise microcapsules having a 2 layer coating. A first coating comprising ethylcellulose and a second coating comprising a layer of an acrylic polymer. This provides thus improved pharmaceutical formulations where the umpleasant taste is masked and that can give an immediate delivery on the stomach. Advantageously the composition can be administered in the form of a suspension for oral administration.

According to the invention the composition comprises
- spherical agglomerates of telithromycin
- ethylcellulose,
- acrylic polymer
- anti-agglomeration agent
In a prefered embodiment, the composition comprises :
- spherical agglomerates of telithromycin
- ethylcellulose,
- acrylic polymer,
- anti-agglomeration agent,
- D(-) N-methylglucamine
- aluminium and magnesium silicate
and optionally agents chosen from suspending agents, aromatization agents, sweetening agents and/or antimicrobial preservation agents.

According to the invention the acrylic polymer can be chosen for instance among Eudragit® E.
For example, the anti-agglomeration agent can be talc.

Among the suspending agents, xanthan gum can be cited for instance, the aromatization agents can be chosen from sugars and for instance maltitol, the antimicrobial preservation agent can be chosen from parabens and the sweetening agent can be chosen for instance from sodium saccharinate.
In a more prefered embodiment the composition comprises :
- spherical agglomerates of telithromycin : 40 to 65%
- ethylcellulose : 8 to 18%,
- acrylic polymer 18 to 35%,
- talc 4.5 to 10%,
- D(-) N-methylglucamine : 0.8 to 1.8%
- aluminium and magnesium silicate : 4 to 9%
and optionally
- xanthan gum : 0.3 to 0.7%
- maltitol : 40 to 90%
- sodium saccharinate : 0.6 to 1.4%
- flavouring agent: 0.6 to 1.4%

More preferably the composition comprises from 45 to 63% of telithromycin agglomerates ; from 9 to 15% of ethylcellulose ; from 18 to 33% of the acrylic polymer; from 5 to 8% of talc, D(-) N-methylglucamine from 0.8 to 1.8%, aluminium and magnesium silicate from 4 to 9% and optionally the above mentioned additives. Also, according to a prefered embodiment of the invention the ratio acrylic polymer /anti-agglomeration agent is advantageously chosen around 2 to 4, preferably 4.

According to the invention the mean size of the telithromycin agglomerates used as starting material is comprised between 30 and 500µm. In a prefered embodiment the mean size is comprised between 60 and 250µm (light scattering analysis).

According to the invention the composition is prepared from telithromycin in the form of spherical agglomerates (as described in WO 01/14393) using 2 main steps of encapsulation :
- first step : microencapsulation with ethylcellulose,
- second step : fluid bed coating of the microcapsules with the acrylic polymer.

In the prefered embodiment where the composition comprises D(-) N-methylglucamine, aluminium and magnesium silicate and optionally suspending agents, aromatization agents, sweetening agents and/or antimicrobial preservation agents these additives are added in a third step of the process after the 2 encapsulation steps. In the more prefered embodiment where the composition comprises D(-) N-methylglucamine, aluminium and magnesium silicate and optionally xanthane gum, maltitol, sodium saccharinate and flavouring agent, these additives are also added in a third step of the process after the 2 encapsulation steps.

The 2 main steps of encapsulation can be advantageously conducted as follows :
- microencapsulation in ethylcellulose can be made by coacervation according to the method described in US 6,139,865 and EP 38585 (incorporated here as a references), with ethylcellulose in cyclohexane and polyethylen wax, followed by separation of the microcapsules from the liquid phase and drying on a fluid bed.
- the encapsulation in an acrylic polymer can be made using fluid bed coating technics, in the presence of an anti-agglomeration agent. The process is carried out in a hydro alcoholic medium (for example in hydro-ethanolic medium).

According to the invention for instance the polyethylen wax can be Epolen®; for instance, the acrylic polymer can be chosen among Eudragit E (Eudragit® E100); the anti-agglomeration agent is preferably talc and more preferably a micronized talc or talc of less than 75µm.

The amount of the primary coating with ethylcellulose is defined such as to get a optimal particule size distribution, residual solvents complying with the regulatory acceptance and such as the microcapsules are suitable to be coated using a fluid bed technic. Usually an amount between 15 and 30% of coating level is suitable ; preferably a coating level between 18 and 25% is advantageously chosen, and more prefered is 18%. The microcapsules prepared with the primary coating comprise 50 to 95 % of telithromycin agglomerates and 5 to 50% of ethylcellulose. After the first coating the mean particule size is acceptable and is for example less than 250µm for more than 90% of the resulting microcapsules.

The secondary coating, made of acrylic polymer in the presence of an anti-agglomeration agent is made in an amount that allows a narrow particule size distribution, together with the properties of satisfactory taste-masking and fast dissolution rate for the preparation of the suspension. Usually an amount between 20 and 45% of coating level is suitable ; preferably a coating level between 25 and 40% is advantageously chosen. After the second coating the mean particule size is less than 300µm for more than 90% of the resulting microcapsules.

According to the invention, it is possible to prepare from the microcapsules, suspensions comprising from 5 to 250 mg/ml and more preferably 100 mg/ml.
The prepared microcapsules have the capability of forming a sufficiently stable suspension, and have a fast dissolution rate : equal or superior to 80% over 2 to 10 minutes and in some preferential aspects of the invention : 90% within 2 mn.

It has been shown that they provide a satisfactory taste masking up to five days after suspension reconstitution.

The following examples illustrate the invention.

### Example 1

The first step of coating was carried out using a 5 liter reactor with a pneumatic stirring, introducing spherical agglomerates of telithromycin with ethylcellulose in cyclohexane and Epolene®, with agitation at a temperature of 80°C, according to the coacervation method described in US 6,139,865.
The resulting microcapsules are sieved

After carrying out the first step, the second coating was performed over 75mn, with using a Glatt GPCG1 fluid bed equipped with Wurster insert (4 "), bottom plate «A» type, filter «T165P» type, 1mm spraying nozzle and adding to 456 g of the first coated microcapsules, Eudragit® E100 10%, talc <75µm 5.0%, ethanol 51%, purified water 34%. The coating layer was applied at a spraying rate of 2.5 to 5.5 g/mn. The temperature of the air was 45 to 52°C and the air speed 1.0 to 1.5 m/s. The atomisation pressure was 1.8 bars. The temperature of the product during the coating was between 30 and 33°C. The coating is performed with an amount of membrane of 25%. After drying on the fluid bed and sieving the corresponding 2 coatings microcapsules are obtained. The resulting composition comprises telithromycin agglomerates 58.5%, ethylcellulose 6.5%, Eudragit® E 23.3%, talc 11.7%. The coated samples were easily dispersible in aqueous medium without any agglomeration or segregation phenomena.

### Example 2

After carrying out a first step according to the example 1, the second coating was performed over 92mn, with using a Glatt GPCG1 fluid bed equipped with Wurster insert (4 "), bottom plate « A » type, filter «T165P» type, 1mm spraying nozzle and adding to 456 g of the first coated microcapsules, Eudragit® E100 10%, talc <75µm 5.0%, ethanol 51%, purified water 34%. The coating layer was applied at a spraying rate of 2.0 to 5.0 g/mn. The temperature of the air was 54 to 55°C and the air speed 1.0 to 1.5 m/s. The atomisation pressure was 1.8 bars. The temperature of the product during the coating was between 30 and 33°C. The coating is performed with an amount of membrane of 5%. After drying on the fluid bed and sieving the corresponding 2 coatings microcapsules are obtained. The resulting composition comprises telithromycin agglomerates 67.5%, ethylcellulose 7.5%, Eudragit® E 16.7%, talc 8.3%. The coated samples were easily dispersible in aqueous medium without any agglomeration or segregation phenomena.

### Example 3

After carrying out a first step according to the example 1 with 3 kg cyclohexane and a coating level of ethylcellulose of 9.5%, the second coating was performed over 92mn, with using a Glatt GPCG1 fluid bed equipped with Wurster insert (4 "), bottom plate « A » type, filter « T165P » type, 1mm spraying nozzle and adding to 456 gof the first coated microcapsules, Eudragit® E100 10%, micronized talc 2.5%, ethanol 52.5%, purified water 35%. In order to avoid any damage to the ethylcellulose coating, the first part of the coating layer was applied at a lower spraying rate : 2.0 to 3.0 g/mn. Then at a spraying rate of 4.8 to 5.8 g/mn. The temperature of the air was 55 to 60°C and the air speed 1.0 to 1.8 m/s. The atomisation pressure was 1.8 bars. The temperature of the product during the first part of the coating was between 33 and 38°C. The temperature of the product during the second part of the coating was between 30 and 32°C. The coating is performed with an amount of membrane of 20.9%. After drying on the fluid bed and sieving the corresponding 2 coatings microcapsules are obtained. The resulting composition comprises telithromycin agglomerates 71.6%, ethylcellulose 7.5%, Eudragit® E 16.7%, talc 4.2%. The coated samples were easily dispersible in aqueous medium without any agglomeration or segregation phenomena. The release is 97 ± 1 after 2 mn.

## Claims

1. Composition for oral administration of telithromycin comprising :
• spherical agglomerates of telithromycin
• ethylcellulose,
• an acrylic polymer
• anti-agglomeration agent
where the composition is in the form of microcapsules having a 2 layer coating.

2. Composition for oral administration of telithromycin, according to claim 1, comprising :
further
• D(-) N-methylglucamine
• aluminium and magnesium silicate
and optionally agents chosen from suspending agents, aromatization agents, sweetening agents and/or antimicrobial preservation agents.

3. Composition for oral administration of telithromycin, according to claim 1, comprising :
• spherical agglomerates of telithromycin : 40 to 65%
• ethylcellulose : 8 to 18%,
• acrylic polymer 18 to 35%,
• talc 4.5 to 10%,
• D(-) N-methylglucamine : 0.8 to 1.8%
• aluminium and magnesium silicate : 4 to 9%
and optionally
• xanthan gum : 0.3 to 0.7%
• maltitol : 40 to 90%
• sodium saccharinate : 0.6 to 1.4%
• flavouring agent: 0.6 to 1.4%
where the composition is in the form of microcapsules having a 2 layer coating.

4. Process for the preparation of a composition as described in anyone of claims 1 to 3, where telithromycin is in the form of spherical agglomerates and is coated using 2 steps of encapsulation, as follows :
• first step : microencapsulation with ethylcellulose,
• second step : fluid bed coating of the microcapsules with the acrylic polymer.

5. Process according to claim 4, where the first coating is made by coacervation in cyclohexane.

6. Process according to claim 4, for the preparation of a composition as described in claim 2, where telithromycin is in the form of spherical agglomerates and is coated using a 2 steps of encapsulation :
• first step : microencapsulation with ethylcellulose,
• second step: fluid bed coating of the microcapsules with the acrylic polymer,
followed by a third step of addition of D(-) N-methylglucamine, aluminium and magnesium silicate, and optionally suspending agents, aromatization agents, sweetening agents and/or antimicrobial preservation agents.

7. Process according to claim 4, for the preparation of a composition as described in claim 3, where telithromycin is in the form of spherical agglomerates and is coated using a 2 steps of encapsulation :
• first step : microencapsulation with ethylcellulose,
• second step : fluid bed coating of the microcapsules with the acrylic polymer,
followed by a third step of addition of D(-) N-methylglucamine, aluminium and magnesium silicate, and optionally xanthane gum, maltitol, sodium saccharinate and flavouring agent.

8. Use of a composition according to anyone of claims 1 to 3 for the taste masking of telithromycin.

9. Composition for oral administration of telithromycin, according to claim1, comprising:
• spherical agglomerates of telithromycin 58.5%
• ethylcellulose 6.5%
• eudragit® E 23.3%
• talc 11.7%.

10. Composition for oral administration of telithromycin, according to claim1, comprising:
• spherical agglomerates of telithromycin 67.5%
• ethylcellulose 7,5%
• eudragit® E 16.7%
• talc 8.3%.

11. Composition for oral administration of telithromycin, according to claim1, comprising:
• spherical agglomerates of telithromycin 71.6%
• ethylcellulose 7.5%
• eudragif® E 16.7%
• talc 4.2%.

## Patentansprüche

1. Zusammensetzung zur oralen Verabreichung von Telithromycin, enthaltend:
• kugelförmige Agglomerate von Telithromycin,
• Ethylcellulose,
• ein Acrylpolymer,
• Antiagglomerationsmittel,
wobei die Zusammensetzung in Form von Mikrokapseln mit einem zweischichtigen Überzug vorliegt.

2. Zusammensetzung zur oralen Verabreichung von Telithromycin nach Anspruch 1, ferner enthaltend:
• D(-)-N-Methylglucamin
• Aluminium- und Magnesiumsilicat
und gegebenenfalls unter Suspendiermitteln, Aromatisierungsmitteln, Süßungsmitteln und/oder antimikrobiellen Konservierungsmitteln ausgewählte Mittel.

3. Zusammensetzung zur oralen Verabreichung von Telithromycin nach Anspruch 1, enthaltend:
• kugelförmige Agglomerate von Telithromycin: 40 bis 65%,
• Ethylcellulose: 8 bis 18%,
• Acrylpolymer: 18 bis 35%,
• Talk: 4,5 bis 10%,
• D(-)-N-Methylglucamin: 0,8 bis 1,8%
• Aluminium- und Magnesiumsilicat: 4 bis 9%
und gegebenenfalls
• Xanthan: 0,3 bis 0,7%
• Maltit: 40 bis 90%
• Natriumsaccharinat: 0,6 bis 1,4%
• Geschmacksverbesserer: 0,6 bis 1,4%
wobei die Zusammensetzung in Form von Mikrokapseln mit einem zweischichtigen Überzug vorliegt.

4. Verfahren zur Herstellung einer Zusammensetzung gemäß einem der Ansprüche 1 bis 3, bei dem Telithromycin in Form von kugelförmigen Agglomeraten vorliegt und folgendermaßen in 2 Verkapselungsschritten überzogen wird:
• erster Schritt: Mikroverkapselung mit Ethylcellulose,
• zweiter Schritt: Überziehen der Mikrokaspseln mit dem Acrylpolymer in der Wirbelschicht.

5. Verfahren nach Anspruch 4, bei dem man den ersten Überzug durch Koazervation in Cyclohexan herstellt.

6. Verfahren nach Anspruch 4 zur Herstellung einer Zusammensetzung gemäß Anspruch 2, bei dem Telithromycin in Form von kugelförmigen Agglomeraten vorliegt und in 2 Verkapselungsschritten überzogen wird:
• erster Schritt: Mikroverkapselung mit Ethylcellulose,
• zweiter Schritt: Überziehen der Mikrokaspseln mit dem Acrylpolymer in der Wirbelschicht, gefolgt von einem dritten Schritt der Zugabe von D(-)-N-Methylglucamin, Aluminium- und Magnesiumsilicat und gegebenenfalls Suspendiermitteln, Aromatisierungsmitteln, Süßungsmitteln und/oder antimikrobiellen Konservierungsmitteln.

7. Verfahren nach Anspruch 4 zur Herstellung einer Zusammensetzung gemäß Anspruch 3, bei dem Telithromycin in Form von kugelförmigen Agglomeraten vorliegt und in 2 Verkapselungsschritten überzogen wird:
• erster Schritt: Mikroverkapselung mit Ethylcellulose,
• zweiter Schritt: Überziehen der Mikrokaspseln mit dem Acrylpolymer in der Wirbelschicht, gefolgt von einem dritten Schritt der Zugabe von D(-)-N-Methylglucamin, Aluminium- und Magnesiumsilicat und gegebenenfalls Xanthan, Maltit, Natriumsaccharinat und Geschmacksverbesserer.

8. Verwendung einer Zusammensetzung nach einem der Ansprüche 1 bis 3 zur Geschmacksmaskierung von Telithromycin.

9. Zusammensetzung zur oralen Verabreichung von Telithromycin nach Anspruch 1, enthaltend:
• kugelförmige Agglomerate von Telithromycin: 58,5%,
• Ethylcellulose: 6,5%,
• Eudragit^{®} E: 23,3%,
• Talk: 11,7%.

10. Zusammensetzung zur oralen Verabreichung von Telithromycin nach Anspruch 1, enthaltend:
• kugelförmige Agglomerate von Telithromycin: 67,5%,
• Ethylcellulose: 7,5%,
• Eudragit^{®} E: 16,7%,
• Talk: 8,3%.

11. Zusammensetzung zur oralen Verabreichung von Telithromycin nach Anspruch 1, enthaltend:
• kugelförmige Agglomerate von Telithromycin: 71,6%,
• Ethylcellulose: 7,5%,
• Eudragit^{®} E: 16,7%,
• Talk: 4,2%.

## Revendications

1. Composition pour administration orale de télithromycine comprenant :
• des agglomérats sphériques de télithromycine
• de l'éthylcellulose,
• un polymère acrylique
• un agent anti-agglomération
la composition étant sous forme de microcapsules enrobées d'un revêtement bicouche.

2. Composition pour administration orale de télithromycine selon la revendication 1, comprenant en outré :
• de la D(-) N-méthylglucamine
• un silicate d'aluminium et de magnésium
et éventuellement des agents sélectionnés parmi les agents de suspension, les agents aromatisants, les agents sucrants et/ou les agents de conservation antimicrobiens.

3. Composition pour administration orale de télithromycine selon la revendication 1, comprenant :
• des agglomérats sphériques de télithromycine : 40 à 65 %
• de l'éthylcellulose : 8 à 18 %
• un polymère acrylique : 18 à 35 %,
• du talc : 4,5 à 10 %,
• de la D(-) N-méthylglucamine : 0,8 à 1,8 %
• un silicate d'aluminium et de magnésium : 4 à 9 %
et éventuellement
• une gomme xanthane : 0,3 à 0,7 %
• du maltitol : 40 à 90 %
• du saccharinate de sodium : 0,6 à 1,4 %
• un agent aromatisant : 0,6 à 1,4 %
la composition étant sous forme de microcapsules enrobées d'un revêtement bicouche.

4. Procédé pour l'élaboration d'une composition telle que décrite dans l'une quelconque des revendications 1 à 3, où la télithromycine est sous forme d'agglomérats sphériques et est enrobée en deux étapes d'encapsulation, comme suit :
• première étape : microencapsulation par de l'éthylcellulose,
• deuxième étape : enrobage à lit fluidisé des microcapsules par le polymère acrylique.

5. Procédé selon la revendication 4, où la première étape d'enrobage est effectuée par coacervation dans le cyclohexane.

6. Procédé selon la revendication 4 pour l'élaboration d'une composition telle que décrite dans la revendication 2, où la télithromycine est sous forme d'agglomérats sphériques et est enrobée en deux étapes d'encapsulation :
• première étape : microencapsulation par de • l'éthylcellulose,
• deuxième étape : enrobage à lit fluidisé des microcapsules par le polymère acrylique,
suivies d'une troisième étape d'addition de D(-) N-méthylglucamine, de silicate d'aluminium et de magnésium, et éventuellement d'agents de suspensions, d'agents aromatisants, d'agents sucrants et/ou d'agents de conservation antimicrobiens.

7. Procédé selon la revendication 4 pour l'élaboration d'une composition telle que décrite dans la revendication 3, où la télithromycine est sous forme d'agglomérats sphériques et est enrobée en deux étapes d'encapsulation :
• première étape : microencapsulation par de l'éthylcellulose,
• deuxième étape : enrobage à lit fluidisé des microcapsules par le polymère acrylique,
suivies d'une troisième étape d'addition de D(-) N-méthylglucamine, de silicate d'aluminium et de magnésium, et éventuellement de gomme xanthane, de maltitol, de saccharinate de sodium et d'un agent aromatisant.

8. Emploi d'une composition selon l'une quelconque des revendications 1 à 3 qui masque le goût de la télithromycine.

9. Composition pour administration orale de télithromycine selon la revendication 1, comprenant :
• des agglomérats sphériques de télithromycine à 58,5 %
• de l'éthylcellulose à 6,5 %
• de l'eudragit® E à 23,3 %
• du talc à 11,7 %.

10. Composition pour administration orale de télithromycine selon la revendication 1, comprenant :
• des agglomérats sphériques de télithromycine à 67,5 %
• de l'éthylcellulose à 7,5 %
• de l'eudragit® E à 16,7 %
• du talc à 8,3 %.

11. Composition pour administration orale de télithromycine selon la revendication 1, comprenant :
• des agglomérats sphériques de télithromycine à 71,6 %
• de l'éthylcellulose à 7,5 %
• de l'eudragit® E à 16,7 %
• du talc à 4,2 %.
